# EUROPEAN PATENT SPECIFICATION

(11) **EP 1 701 166 B1**
(45) Date of publication and mention of the grant of the patent: **06.01.2010**
(21) Application number: 05077320.9
(22) Date of filing: 11.10.2005
(51) Int. Cl.: G01N 33/68

(54) **Phosphorylated SP1 as a marker in diagnosis of non-alcoholic steatohepatitis (nash) and target in drug screening for nash**
Phosphorylierte SP1 als Marker bei der Diagnose nichtalkoholischer Steatohepatitis (NASH) und Ziel beim Drogenscreening auf NASH
SP1 phosphorylé comme marqueur pour le diagnostic de stéato-hépatite non alcoolique (nash) et objectif pour le criblage de drogue pour nash

(30) Priority: 11.03.2005 EP 05075602
(43) Date of publication of application: 13.09.2006
(73) Proprietor: One Way Liver Genomics, S.L., 48160 Vizcaya (ES)
(72) Inventor: Mato de la Paz, José Maria, 48160 Derio - Vizcaya (ES)
(74) Representative: ABG Patentes, S.L.

(56) References cited:
- WO-A-02/066071
- WO-A1-2004/055520
- VERRECCHIA F ET AL: "Blocking Sp1 transcription factor broadly inhibits extracellular matrix gene expression in vitro and in vivo: Implications for the treatment of tissue fibrosis" JOURNAL OF INVESTIGATIVE DERMATOLOGY, NEW YORK, NY, US, vol. 116, no. 5, May 2001 (2001-05), pages 755-763, XP002228966 ISSN: 0022-202X
- IHN HIRONOBU ET AL: "Increased phosphorylation of transcription factor Sp1 in scleroderma fibroblasts: Association with increased expression of the type I collagen gene" ARTHRITIS AND RHEUMATISM, vol. 43, no. 10, October 2000 (2000-10), pages 2240-2247, XP002374830 ISSN: 0004-3591
- BLACK ADRIAN R ET AL: "Growth/cell cycle regulation of Sp1 phosphorylation" JOURNAL OF BIOLOGICAL CHEMISTRY, vol. 274, no. 3, 15 January 1999 (1999-01-15), pages 1207-1215, XP002374831 ISSN: 0021-9258
- LEGGETT R W ET AL: "Spi is phosphorylated and its DNA binding activity down-regulated upon terminal differentiation of the liver" JOURNAL OF BIOLOGICAL CHEMISTRY, AMERICAN SOCIETY OF BIOLOCHEMICAL BIOLOGISTS, BIRMINGHAM,, US, vol. 270, no. 43, 27 October 1995 (1995-10-27), pages 25879-25884, XP002952975 ISSN: 0021-9258
- LU S C ET AL: "Methionine adenosyltransferase 1A knockout mice are predisposed to liverinjury and exhibit increased expression of genes involved in proliferation" PROCEEDINGS OF THE NATIONAL ACADEMY OF SCIENCES OF USA, NATIONAL ACADEMY OF SCIENCE, WASHINGTON, DC, US, vol. 98, no. 10, 8 May 2001 (2001-05-08), pages 5560-5565, XP002980762 ISSN: 0027-8424
- CHU S ET AL: "Sp1: Regulation of gene expression by phosphorylation" GENE: AN INTERNATIONAL JOURNAL ON GENES AND GENOMES, ELSEVIER, AMSTERDAM, NL, vol. 348, 28 March 2005 (2005-03-28), pages 1-11, XP004803928 ISSN: 0378-1119

## Description

### FIELD OF THE INVENTION

The invention refers, in general, to *in vitro* diagnosis of non-alcoholic steatohepatitis (NASH); more specifically to the early diagnosis of NASH or to confirm the disease, based on an overexpression of phosphorylated Spl transcription factor in liver cells. The invention also refers to monitor the effect of therapy administered to an individual with NASH. Additionally, the invention refers to screen, seek, identify, develop and evaluate the efficacy of compounds for the prevention and/or treatment of NASH in an attempt to develop new medicinal products as well as agents that inhibit the expression and/or activity of Sp1 in liver, and/or the effects of its expression.

### BACKGROUND OF THE INVENTION

Non-alcoholic steatohepatitis (NASH) is a progressive disease of the liver of unknown ethiology characterized histologically by fatty acid accumulation, hepatocyte damage and inflammation resembling alcoholic hepatitis. NASH is a critical stage in the process that spans from hepatic steatosis to cirrhosis and liver failure. Obesity and type-2 diabetes are associated to NASH. Since the prevalence of these diseases is increasing, the prevalence of NASH is also expected to increase and therefore, this disease has become an emerging public issue in the United States [Reid AE. Nonalcoholic steatohepatitis. Gastroenterology 2001;121:710-723] as well as in other countries [Farell GC. Non-alcoholic steatohepatitis: what is it, and why is it important in the Asia-Pacific region? J Gastroenterol Hepatol 2003;18:124-138].

NASH is thought to arise from the interaction of many different genes and lifestyle factors. Mitochondrial impairment, oxidative stress and metabolic deregulation, have all been involved in the pathogenesis of stcatohepatitis.

As it is true for other complex diseases, the genetic factors contributing to the development of NASH may be more readily identified by combining studies in patients with NASH and in animal models of the disease. One of these models is *M*Λ*T1*Λknockout (MAT1A-KO) mice. These mice spontaneously develop NASH and hepatocellular carcinoma at about 8 and 15 months of age, respectively [Lu SC et al. Proc. Natl. Acad. Sci. USA 98:5560-5565 (2001); Martinez-Chantar ML et al. FASEB J 2002.

*MAT1A* gene encodes for methionine adenosyltransferase I and III, the main enzymes responsible of S-adenosylmethionine (SAMe) synthesis in liver. Earlier studies concluded that patients with liver cirrhosis and alcoholic hepatitis are deficient in SAMe synthesis; and that treatment with SAMe improves survival in patients with alcoholic liver cirrhosis.

Early diagnosis of NASH has been held back by the lack of reliable early markers of NASH development. Identification of genes and proteins expressed differentially in NASH with potential as biological markers or therapeutic targets could lead to the development of new tools for the diagnosis, prognosis and treatment of this disease.

A method for the diagnosis of NASH by using molecular markers based on the proteomic determination of a set of proteins detected in liver tissue samples has been disclosed (WO2004/055520).

Another method for the diagnosis of NASH based on the identification of a cluster of 85 discriminative early gene markers specific of NASH in liver tissue samples (thus, constituting what has been named the "genomic signature or fingerprint of NASH") has been also described by the Applicant (EP 04103540.3). The technique used in that case combines liver samples from MAT1A-KO mice and from patients with early-stage NASH with bioinformatic and statistical methods to analyze the data generated from genome-wide expression profiling of the mentioned liver samples, thus allowing the identification of a cluster of discriminative early gene markers of steatohepatitis in mice and humans. Among the genes comprising the genomic signature of NASH there are enzymes (the majority being hydrolases, transferases and oxidoreduetases), ligand-binding genes (heavy metal binding, nucleotide binding, protein binding, receptors and transcription factors); transporters (carbohydrate, electron transporter, and protein transporters); apoptosis regulators; chaperones; blood coagulation factors; and several genes with unknown function.

Looking for the presence of consensus sequences for vertebrate transcription factors among the promoters of the genes listed in Table 1 (EP 04103540.3), the inventors have now, surprisingly, found that only Spl transcription factor was present significantly in the promoters of a great number of the genes listed in said Table 1, more than would be expected by chance, suggesting that activation of Spl may be involved in the underlying mechanisms that lead to NASH.

Spl was one of the first eukaryotic transcription factors to be identified and cloned as a factor binding the SV40 early promoter (Dynan and Tjian, Cell 35:79-87, 1983). It is the founding member of a family of proteins with highly homologous zinc-finger domains in the C-terminal region that bind GC or GT boxes, while the glutamine rich domains in the N-terminus are essential for transcriptional activation. Spl activates transcription by association with one of the co-activators associated with the TATA binding protein (TBP) in the TFHD complex. Other suggested roles for Spl in nuclear processes include remodeling of chromatin structures and maintenance of methylation-free CpG islands. Therefore, Spl is fundamental for the establishment of transcriptional competence, in addition to its role as a transcription factor. In a majority of promoters containing Spl binding sites, Spl provides a basal level of transcription. It plays an important role in the expression of numerous elements of the cell-cycle machinery, such as cyclins, Rb-like proteins, and E2F. Targeted disruption of the mouse Spl gene has shown that Spl is critical for normal embryogenesis. Spl (-/-) embryos are severely retarded in their development and display a marked heterogeneity in their phenotype. Interestingly, inactivation of the Spl gene is compatible with a certain degree of cell growth and differentiation, and the expression of various putative target genes, including that of certain cell cycle-related genes, was not altered in Spl (-/-) embryos. Also, CpG islands remained methylation free and active chromatin was formed at the globin loci. This may occur possibly because other members of the Spl family partially compensate for the absence of Spl, thereby ameliorating the Spl knockout. Spl is involved in the basal expression of extracellular matrix (ECM) genes and is important in fibrotic processes. Thus, blocking Spl inhibits ECM gene expression what can be used in the treatment of fibrotic disorders (WO 02/066701).

### BRIEF DESCRIPTION OF DRAWINGS

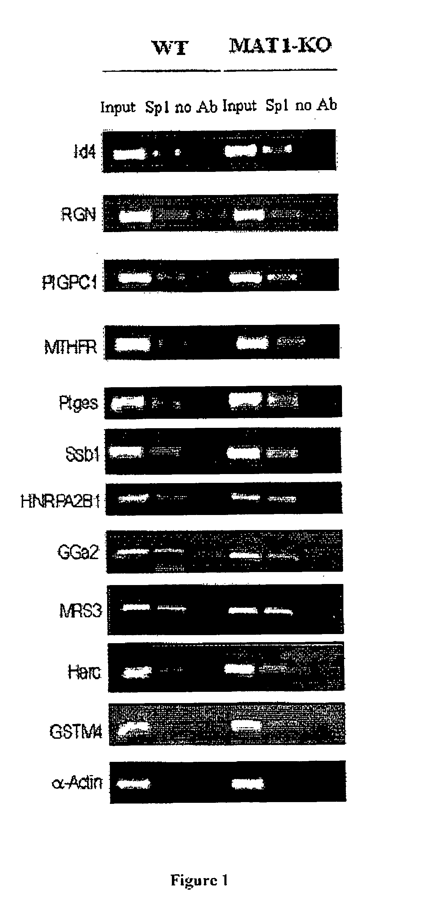

Figure 1 shows the results of an *in vivo* binding analysis of Spl to selected gene promoters. Immunoprecipitation of formaldehyde-crosslinked chromatin was carried out in liver tissue samples from control mice (Wild Type, WT) and in liver tissue samples from MAT1A-KO mice by using an anti-Spl antibody (Example 1). Immunoprecipitates were aliquoted and subsequently analyzed by PCR with primers specific for each gene promoter. In each case, a sample of total chromatin (Input) was included in the PCR reactions. No Ab: No antibody. Spl: Immunoprecipitation fraction. α-Actin promoter was included as a negative control.

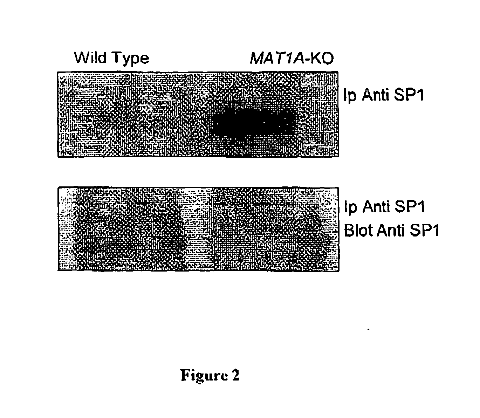

Figure 2 shows Spl phosphorylation in mice liver tissue. Total crude extract from WT and MAT1A-KO mice liver was inmunoprecipitated (Ip) with an anti-Spl antibody and screened for the presence of phosphoserine with an anti-phosphoserine antibody (upper panel) or screened for total Spl content with an antibody against Sp1 (lower panel). The results show that the Spl phosphorylation state is increased in liver tissue samples from MAT1A-KO mice versus the Spl phosphorilation state in liver tissue samples from WT mice. Lower panel shows that the amount of total Sp1 content screened with an anti-Spl antibody is the same in MAT1A-KO and WT mice in the total crude extract.

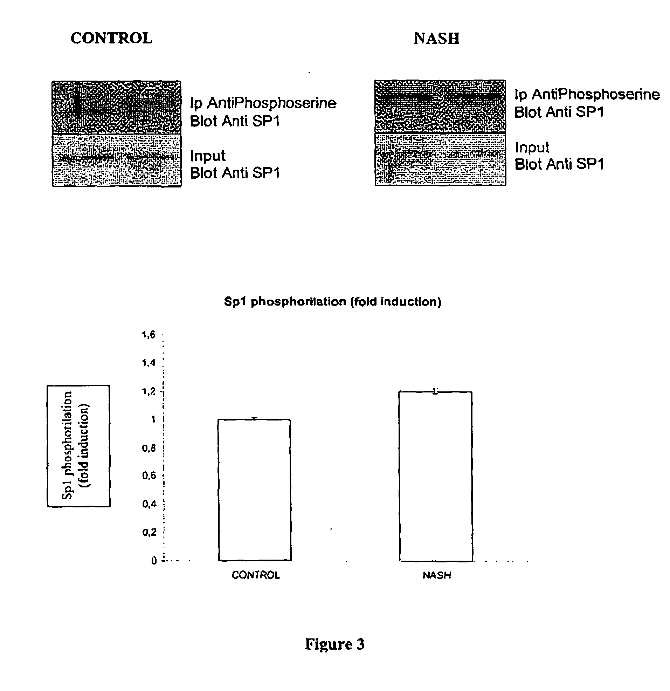

Figure 3 shows Spl phosphorylation in human liver tissues. Total crude extract from human liver tissues from control subjects (CONTROL) and NASH diagnosed subjects (NASH) was immunoprecipitated (Ip) with an anti-phosphoscrine antibody and screened for the presence of Spl with an anti-Spl antibody (upper panel) or was screened for total Spl content by using an anti-Spl antibody (lower panel) (Figure 3A). Densitometric changes are shown in Figure 3B, expressed as fold of induction of Spl phosphorylation over control sample value. Differences between NASH and control were statistically significant, p<0.05.

### DETAILED DESCRIPTION OF THE INVENTION

### Definitions

In order to facilitate the comprehension of the present patent application we list below the meaning of some terms and expressions within the context of the invention.

The term "subject" refers to all animals classified as mammals and includes, but is not restricted to, domestic and farm animals, primates and humans. The subject is preferably a male or female human of any age or race.

The term "NASH" refers to non-alcoholic steatohepatitis.

The term "gene" refers to a region of a molecular chain of deoxyribonucleotides that encodes a protein and may represent a portion of a coding sequence or a complete coding sequence.

The term "DNA" refers to deoxyribonucleic acid. A DNA sequence is a sequence of deoxyribonucleotides.

The term "RNA" refers to ribonucleic acid. A RNA sequence is a sequence of ribonucleotides.

The term "mRNA" refers to messenger ribonucleic acid, which is the fraction of total RNA which is translated into protein.

The term "cDNA" refers to a nucleotide sequence complementary to a sequence of mRNA.

The tenn "nucleotide sequence" refers either to a sequence of ribonucleotides (RNA) or a sequence of deoxyribonucleotides (DNA).

The term "protein" refers to at least one molecular chain of amino acids linked through either covalent or non-covalent bonds. The term includes all forms of post-translational protein modifications, for example glycosylation, phosphorylation or acetylation.

The term "antibody" refers to a glycoprotein that exhibits a specific binding activity for a target molecule called an "antigen". The term "antibody" includes monoclonal and polyclonal antibodies, either intact or fragments derived from them; it also includes human antibodies, humanised antibodies and antibodies of non-human origin. "Monoclonal antibodies" is a homogeneous, highly specific antibody population that can bind to a single antigenic site or "determinant" on the target molecule. "Polyclonal antibodies" is a heterogeneous antibody population that can bind to multiple antigenic sites or "determinants" of the target molecule.

The term "cpitopc" refers to an antigenic determinant of a protein recognised by a specific antibody. An epitope may consist of a contiguous stretch of amino acids (linear epitope), of non-contiguous amino acids that are brought into proximity with one another by virtue of the three dimensional folding of the polypeptide chain (discontinuous epitopes), of post-translational modifications of a protein or of a combination thereof.

The term "therapeutic target" refers to nucleotide or peptide sequences against which a drug or therapeutic compound can be designed and applied.

The term "antagonist" refers to any molecule that inhibits the biological activity of the antagonised molecule. Examples of antagonist molecules include proteins, peptides, variations of natural peptide sequences, small organic molecules (usually molecules with a molecular weight under 500 Daltons), etc.

### Sp1 as a marker of NASH and/or target for drug screening

The invention is based on the finding that the regulation of the proteins responsible for Spl phosphorylation and the concentration (level) of phosphorylated Sp1 protein are increased in liver tissue samples from NASH patients but not in liver tissue samples from NASH free subjects.

As it has been previously mentioned, the inventors, looking for the presence of consensus sequences for all vertebrate transcription factors listed in the Gene Regulation database (http://www.gene-regulation.com/) among the promoters of the genes listed in Table 1 (EP 04103540.3), found that only Sp1 transcription factor was present significantly in the promoters of 34 of the genes listed in said Table 1, suggesting that phosphorylation of Spl may be involved in the underlying mechanisms that lead to NASH. Spl is among the transcription factors that are redox-activated. Since 10 of the NASH-associated genes identified here encode for proteins localized in the mitochondria and MAT1A-KO mice have mitochondrial dysfunction and oxidative stress, it seems that activation of Sp1 may be involved in the underlying mechanisms that lead to NASH.

Table 1 includes the 85 discriminative gene markers specific of NASH in human liver tissue samples which constitute the genomic signature or fingerprint of NASH in humans, disclosed in Table 1 of EP 04103540.3. Said Table I also includes an indication of the genes which promoters contain a Spl binding site.

**Table I**

| **NAME** | **DESCRIPTION** | **HE** | **SP1** |
|---|---|---|---|
| ***Apoptosis regulador*** | | | |
| Bag1 | Bcl2-associated athanogene 1 | + | Y |
| ***Chaperone*** Bag1 | Bcl2-associated athanogene 1 | + | Y |
| Wbser18 | Similar to syntaxin 1A (brain) | + | |
| ***Defense***/***immunity protein***_*Blood coagulation factor* | | | |
| F11 | Coagulation factor X1 | - | |
| ***Enzyme***_*Hydrolase* | | | |
| 1300019N10Rik | Clone:1300019N10, hypothetical protein | - | |
| 2310010M10Rik* | Similar to cytidine and dCMP deaminase domain containing 1 (NYD-SP15) | + | |
| Ahcyl1 | S-adenosylhomocysteine 1 hydrolase-like | - | |
| AI507170* | Similar to platelet-activating factor acetylhydrolase 2, 40kDa (PAFAH2) | + | |
| Apex1 | Apurinicapyrimidinic endonuclease | + | |
| BC026374* | Similar to carboxylesterase 2 (CES2) | - | |
| Dnasel13 | Deoxyribonuclease 1-like 3 | + | |
| Es10 | Esterase 10 | - | |
| F11 | Coagulation factor XI | - | |
| Gna14 | Guanine nucleotide binding protein, alpha 14 | - | |
| Pgls | Similar to 6-phosphogluconolactonase | - | |
| Psma6 | Proteasome (prosome, macropain) subunit, alpha type 6 | + | |
| ***Enzyme***_*Isomerase* | | | |
| Hpgd | Similar to hydroxyprostaglandin dehydrogenase 15-(NAD) | + | |
| Ptges | Prostaglandin E synthase | - | Y |
| ***Enzyme***_*Kinase* | | | |
| | Similar to cytidine and dCMP deaminase | + | |
| 2310010M10Rik* | domain containing 1 (NYD-SP15) | | |
| Frap1 | FK506 binding protein 12-rapamycin associated protein 1 | - | |
| Galk1 | Galactokinase | + | Y |
| Pip5k1b | Similar to phosphatidylinositol-4- | + | |
| | phosphate 5-kinise, type 1 beta | | |
| ***Enzyme***_*Ligase* | | | |
| Asns ***Enzyme***_*Lyase* | Asparagine synthetase | - | Y |
| Aco2 | Similar to Aconitase 2, mitochondrial | + | Y |
| Apex1 | Apurinicapyrimidinic endonuclease | + | |
| Umps | Uridine monophosphate synthetase | + | |
| ***Enzyme***_*Monooxygenase* | | | |
| Cyp2a5 | Cytochrome P450, 2a5 Cytochrome P450, subfamily IVF, | - | |
| Cyp4fl4 | polypeptide 14 (leukotriene B4 omega hydroxylase) | *** | |
| ***Enzyme***_*Oxidoreductase* | | | |
| Cyp2a5 | Cytochrome P450, 2a5 Cytochrome P450, subfamily IVF, | - | |
| Cyp4fl4 | polypeptide 14 (leukotriene B4 omega hydroxylase) | *** | |
| Deer1 | 2,4-dienoyl CoA reductase 1, mitochondrial | + | |
| Gpd1 | Glycerol phosphate dehydrogenase 1, cytoplasmic adult | + | Y |
| Hao1 Hpgd | Hydroxyacid oxidase 1, liver Similar to hydroxyprostaglandin | + | |
| | dehydrogenase 15-(NAD) Similar to 5,10- | | |
| Mthfr | methylenetetrahydrofolate reductase (NADPH) | - | Y |
| ***Enzyme***_*Transferase* | | | |
| 4930479F15Rik* | Similar to hydroxyacyl-Coenzyme A dehydrogenase/3-ketoacyl-Coenzyme A | + | Y |
| | thiolase/enoyl-Coenzyme A hydratase, beta subunit (HADHB) | | |
| Acat1 | Similar to Acetyl-Co A acetyltransferase | + | Y |
| ACTL | Acetyl CoA transferase-like protein (ACAT2) | + | Y |
| Cmas | CMP-N-acetylneuraminic acid synthetase Similar to Dihydrolipoamide S- | + | |
| Dlat | acetyltransferase (E2 component of pyruvate dehydrogenase complex) | + | Y |
| Frap1 | FK506 binding protein 12-rapamycin associated protein 1 | - | |
| Galk1 | Galactokinase | + | Y |
| Gstm2 | Glutathione S-transferase, mu 2 | - | |
| Gstm4 | Glutathione transferase GSTM7-7 | - | Y |
| Pip5k1b | Similar to phosphatidylinositol-4- | + | |
| | phosphate 5-kinase, type 1 beta Similar to serine | | |
| Shmt2 | hydroxymethyltransferase 2 (mitochondrial) | - | |
| Umps | Uridine monophosphate synthetase | + | |
| ***Enzyme regulator*** _*Enzyme activator* | | | |
| 1190002H23Rik* | Similar to response gene to complement 32 (RGC32) | + | |
| ***Enzyme regulator***_*Kinase regulador* | | | |
| 1190002H23Rik* | similar to response gene to complement 32 (RGC32) | + | |
| ***Ligand binding or carrier*** | | | |
| Mrs3/4-pending | Similar to putative mitochondrial solute carrier (MRS3/4) | + | Y |
| S1c25a5 | Solute carrier family 25 (mitochondrial carrier; adenine nucleotide translocator) | + | |
| ***Ligand binding or carrier***_*Calcium binding* | | | |
| 1110030N17Rik* | Similar to mitochondrial Ca2+- dependent solute carrier (MCSC) | - | |
| Cab39 | Calcium binding protein, 39 | + | Y |
| Rgn | Regucalcin | + | Y |
| S100a10 | S100 calcium binding protein A10 (calpactin) | + | |
| ***Ligand binding or carrier**_Heavy metal binding* | | | |
| 2310010M10Rik* | Similar to cytidine and dCMP deaminase domain containing 1 (NYD-SP15) | + | |
| Aco2 | Similar to Aconitase 2, mitochondrial | + | Y |
| ***Ligand binding or carrier***_*Nucleic acid binding* | | | |
| 2810480G15Rik | Expressed sequence AI503051 | - | Y |
| 4633401C23Rik* | Similar to Zinc finger protein 565 (FLJ36991) | - | |
| Apex1 | Apurinicapyrimidinic endonuclease | + | |
| Dnasel13 | Deoxyribonuclease 1-like 3 TAF4A RNA polymerase II, TATA box | + | |
| LOC228980 | binding protein (TBP)-associated factor 135 kDa | - | Y |
| Pparg | Peroxisome proliferator activated receptor gamma | + | |
| Rbpms | RNA binding protein gene with multiple splicing | + | |
| ***Ligand binding** or **carrier***_*Nucleotide binding* | | | |
| 2610313E07Rik* | Similar to ADP-ribosylation factor-like 10C (FLJ10702) | + | |
| Galk1 | Galactokinase | + | Y |
| Gna14 | Guanine nucleotide binding protein, alpha 14 | - | |
| Gpd1 | Glycerol phosphate dehydrogenase 1, cytoplasmic adult | + | Y |
| Slc2a1 | Solute carrier family 2 (facilitated glucose transporter), member 1 | - | |
| ***Ligand binding or carrier***_*Protein binding* | | | |
| 1190002H23Rik* | Similar to response gene to complement 32 (RGC32) | + | |
| Bagl | Bcl2-associated athanogene 1 | + | Y |
| Cdc371 | Cell division cycle 37 homolog (S. cerevisiae)-like (HARC) | + | Y |
| Fegrt | Fe receptor, IgG, alpha chain transporter | + | Y |
| ***Ligand binding or carrier**_Transcription factor* | | | |
| LOC228980 | TAF4A RNA polymerase II, TATA box binding protein (TBP)-associated factor 135 kDa | - | Y |
| Pparg | Peroxisome proliferator activated receptor gamma | + | |
| ***Signal transducer*** | | | |
| Gna14 | Guanine nucleotide binding protein, alpha 14 | - | |
| ***Signal transducer***_*Receptor* | | | |
| 5031409J19Rik | RIKEN cDNA 5031409J19 gene | + | |
| Fegrt | Fe receptor, IgG, alpha chain transporter | + | Y |
| Pparg | Peroxisome proliferator activated receptor gamma | + | |
| ***Translation regulador*** | | | |
| Pparg | Peroxisome proliferator activated receptor gamma | + | |
| ***Transporter*** | | | |
| C730032N17Rik | Similar to solute carrier family 17 | - | |
| Slc25a5 | (sodium phosphate), member 2 Solute carrier family 25 (mitochondrial carrier; adenine nucleotide translocator) | + | |
| ***Transporter***_*Carbohydrate transporter* | | | |
| Slc2a1 | Solute carrier family 2 (facilitated glucose transporter), member 1 | - | |
| ***Transporter***_*Carrier* | | | |
| Slc2a1 | Solute carrier family 2 (facilitated glucose transporter), member 1 | - | |
| ***Transporter***_*Electron transporter* | | | |
| Hpgd | Similar to hydroxyprostaglandin dehydrogenase 15-(NAD) | + | |
| Haol | Hydroxyacid oxidase 1, liver | + | |
| ***Transporter***_*Protein transporter* | | | |
| Snx5 | Sorting nexin 5 | - | |
| ***Unkown molecular function*** | | | |
| 0610010K14Rik | Myb-like DNA-binding domain | + | Y |
| 1110007C05Rik* | containing protein Similar to hypothetical transmembrane protein SBBI54 | - | |
| 1810018L08Rik | Socs-5 | + | |
| 2010313D22Rik | RIKEN cDNA 2510002A14 gene | - | |
| | 2400006A19Rik* Similar to proteasome regulatory particle subunit P44s10 | + | |
| 2810428F02Rik* | Similar to transmembrane protein 19 (FLJI0936) | + | |
| 3110005G23Rik | Similar to RIKEN cDNA 3110005G23 gene, MGC:28055 | - | |
| 4930422J18Rik* | Similar to SPRY domain-containing SOCS box protein SSB-1 | + | Y |
| 9130414A06Rik | Heterogeneous nuclear ribonucleoprotein A2B1 (HNRPA2B1) | + | Y |
| Bcl7c* | Similar to glutamate dehydrogenase 1 (GLUD1) | + | Y |
| Cd8l | CD 81 antigen | + | Y |
| D530030D03Rik* | Similar to HSPC163 protein | + | |
| Duajd1 | DnaJ (Hsp40) homolog, subfamily D, member 1 | | |
| Fbxl10 | F-box protein FBL10 Homolog to ADP-ribosylation factor | - | Y |
| Gga2 | binding protein GGA2 (Gamma-adaptin related protein 2) | - | Y |
| Idb4 | Inhibitor of DNA binding 4 (ID4) | - | Y |
| Il17r1 | Interleukin 17 receptor-like | + | Y |
| LOC216892* | Similar to MYB binding protein (P160) | - | Y |
| | la (MYBBP1A) P53 apoptosis effector related to Pmp22 | | |
| Perp-pending | (PIGPC1) | + | Y |
| Rpa3 | Similar to replication protein A3, 14kDa | + | |
| Rtn4 | Homolog to GLUT4 VESICLE 20 KDA | + | Y |
| SELENBP1** | PROTEIN Similar to Chromosome 14 open reading | + | |
| Sgce | frame 52 Sarcoglycan, epsilon | + | Y |
| Terel-pending* | Similar to transitional epithelia response protein (TERE1) | - | Y |
| Tsap6-pending* | Similar to Dudulin 2 (TSAP) | - | Y |
| Ttc3 | Tetratricopcptidc repeat domain | - | |

| | | | |
|---|---|---|---|
| HE - Human expression Sp1= "Y" means presence of the consensus sequence to bind Sp1 | | | |

Since it has been shown that genes that are co-expressed in multiple microarray data sets have functional relationships (Lee et al., Co-expression analysis of human genes across many microarray data sets. Genome Res 14:1085-1094 (2004)), further analysis of the genes identified in the genomic signature of NASH should give greater insight in the underlying mechanism of NASH. In order to identify *in vivo* Sp1 target sequences, a chromatin immunoprecipitation (ChIP) assay was carried out (Example 1). Briefly, liver chromatin from wild type (WT) and MAT1A-KO mice was immunoprecipitated with an anti-Spl antibody and the genomic DNA was subsequently used for PCR amplification with specific primers for those genes identified whose promoters had a potential Spl binding site. The results (Figure 1) show that Sp1 binds to the promoters of said 11 genes, being the binding of Spl to the promoters of said 11 genes enhanced in MAT1A-KO mice liver as compared to WT mice liver. The expression of 9 of said 11 genes (ID4, PIGPC1, MTHFR, PTGES, SSB1, HNRPA2B1, MRS3, HARC and GSTM) was enhanced in MAT1A-KO mice liver as compared to the expression thereof in WT mice liver. However, the expression levels of 2 genes (RGN and GGa2) was similar in livers of WT and MAT1 A-KO mice. Furthermore, Sp1 phosphorylation seems to be mediating the activation of said genes (Example 2) because Sp1 protein is found in a higher phosphorylated state in liver tissue samples from MAT1A-KO mice than in liver tissue samples from WT mice (Figure 2).

### Diagnostic and prognostic methods

As it has been previously mentioned, the present invention is based on the finding that the phosphorylation state of Sp1 protein is increased in liver tissue samples from NASH patients but not in liver tissue samples from NASH free subjects. In fact, subjects diagnosed with NASH present, in liver tissue samples, high levels of phosphorylated Sp1 relative to the levels in liver samples from NASH free subjects (i.e., subjects without a clinical history of NASH or control subjects).

Accordingly, the evaluation and comparison of the levels of phosphorylated Sp1 protein in liver tissue samples can be diagnostic of NASH. For example, an elevated level of phosphorylated Sp1 protein in a liver tissue sample is indicative of NASH Therefore, the above mentioned finding can be used in one or more of the following methods: diagnostic assays, monitoring clinical trials and screening assays as further described herein.

Therefore, in an aspect, the invention relates to an *in vitro* method to detect NASH in a subject, or to monitor the effect of the therapy administered to a subject with said condition, hereinafter referred to as the method of the invention, which comprises:
a) quantifying the level of phosphorylated Sp1 protein in a liver tissue sample from said subject, and;
b) comparing said level to that of a control sample;
wherein an increase in said level relative to that of the control is indicative of NASH, i.e., it is an indication that said subject is suffering from NASH. The level of phosphorylated Sp1 protein in a liver tissue sample as compared to that of a liver tissue control sample can be useful for monitoring the effect of the therapy administered to a subject with said condition.

In order to carry out the method of the invention, a sample is obtained from the subject under study. In a particular embodiment, the sample is a liver tissue sample, which can be obtained by conventional methods, e.g., by biopsy, by using methods well known to those of ordinary skill in the related medical arts. Methods for obtaining the sample from the biopsy include gross apportioning of a mass, or microdissection or other art-known cell-separation methods. Samples can be obtained from subjects previously diagnosed or not with NASH, or from subjects who are receiving or have previously received anti-NASH treatment.

Because of the variability of the cell types in diseased-tissue biopsy material, and the variability in sensitivity of the diagnostic methods used, the sample size required for analysis may range from 1, 10, 50, 100, 200, 300, 500, 1,000, 5,000, 10,000, to 50,000 or more cells. The appropriate sample size may be determined basted on the cellular composition and condition of the biopsy. The standard preparative steps for said determination are well known to one of ordinary skill in the art.

In a particular embodiment, with the aim of quantifying the phosphorylation of Sp1 protein, the method of the invention comprises (i) contacting the protein extract extracted from the sample with a composition comprising one or more antibodies specific for one or more phospho-serine residues in phosphorylated Sp1 protein, and (ii) quantifying the complexes Sp1-antibodies formed. There is a wide range of immunological assays (immunoassays) available to detect and quantify the formation of specific antigen-antibody complexes; a number of protein-binding assays, competitive and non-competitive, have been previously described, and several of these are commercially available. Hence, the amount of phosphorylated Sp1 protein can be quantified by means of specific antibodies to phosphorylated Spl, i.e., antibodies which recognize (or bind) phosphorylated Spl, such as, phospho-specific antibodies, antibodies which recognize any epitope on phosphorylated Sp1 protein, for example, antibodies that recognize the binding between the phosphorylation site (e.g., a serine, threonine or tyrosine residue) and a phosphorus-containing moiety, or any epitope, for example, a conformational epitope, generated in the phosphorylated Sp1 protein as a result of the phosphorylation of Sp1. Said antibodies can be in the form of monoclonal antibodies, polyclonal antibodies, intact or recombinant fragments of antibodies, combibodies and Fab or scFv of antibody fragments. These antibodies can be human, humanised or non-human in origin. The antibodies used in these assays can be labelled or unlabelled; the unlabelled antibodies can be used in agglutination assays; the labelled antibodies can be used in a wide range of assays. Antibody labels include radionucleotides, enzymes, fluorophores, chemiluminescent reagents, enzyme substrates or cofactors, enzyme inhibitors, particles, colorants and derivatives. There is a wide variety of assays well known to those skilled in the art that can he applied to the present invention, which use unlabelled antibodies as primary reagents and labelled antibodies as secondary reagents. These techniques include but are not limited to Western-blot or Western transfer, ELISA (Enzyme-linked immunosorbent assay), RIA (Radioimmunoassay), Competitive EIA (Competitive enzyme immunoassay), DAS-ELISA (Double antibody sandwich-ELISA), immunocytochemical and immunohistochemical techniques, techniques based on biochips or protein microarrays that use specific antibodies, and colloidal precipitation-based assays in formats such as dipsticks. Other techniques to detect and quantify the phosphorylation of Sp1 protein are affinity chromatography, ligand binding assays and lectin binding assays.

The final step of the method of the invention involves comparing the level of phosphorylated Sp1 protein quantified in a liver tissue sample from the subject under study to the level of phosphorylated Sp1 protein in a liver tissue control sample (i.e., baseline level or reference value). The levels of phosphorylated Sp1 protein in liver tissue control samples can be determined by measuring levels of phosphorylated Sp1 protein in a liver tissue sample from NASH free subjects (i.e., control subjects with respect to NASH). An increase in the level of phosphorylation of Sp1 protein in a liver tissue sample from the subject under study relative to the level of Sp1 protein in a liver tissue control sample is indicative of NASH, i.e., it is an indication that said subject is suffering from NASH. Further, the level of phosphorylated Sp1 protein in a liver tissue sample as compared to that of a liver tissue control sample can also be useful for monitoring the effect of the therapy administered to a subject with said condition.

The method of the invention, based on the measurement of the level (concentration) of phosphorylated Sp1 protein in liver tissue samples is highly sensitive and specific.

In other aspect, the invention refers to the use of a phosphorylated Sp1 protein to *in vitro* detect or diagnose NASH in a subject, or to monitor the effect of the therapy administered to a subject with said NASH.

### Drug screening assays

The invention also provides a method (also referred to herein as a "screening assay") for identifying candidate agents (e.g., small molecules, nucleic acids, peptides, antibodies, peptidomimetics or other drugs) which have a modulatory effect on, for example, Sp1 expression or on Sp1 biological activity.

Therefore, another aspect of the invention relates to an *in vitro* method for identifying and/or evaluating the efficacy of a potentially therapeutic agent against NASH, which comprises:
a) contacting a culture of liver cells with a test compound under the appropriate conditions and for the required period of time for them to interact;
b) determining the level of phosphorylated Sp1 protein;
c) comparing staid level obtained in step b) to that of a control culture of liver cells lacking said test compound; and
d) selecting a test compound which causes said level of step b) to decrease.

The selected compounds can be used, for example, for further testing as a potential agent for the prophylactic and/or therapeutic treatment of NASH.

The determination (detection and quantification) of the levels of phosphorylated Sp1 protein can be performed in a similar manner to that described in connection with the method of the invention.

Since phosphorylated Sp1 activates the expression of some genes, and increased levels of phosphorylated Sp1 protein are found in liver tissue samples from subjects suffering from NASH an agent that decreases the levels of phosphorylated Sp1 protein or that reverses the effects of increased levels thereof, for example, by inhabiting or reducing the phosphorylation of Sp1 protein, becomes a candidate for NASH therapy (prophylaxis and/or treatment). Agents that arc found by using the screening assays provided by the instant invention to be capable of decreasing Sp1 activity by at least 5%, more preferably by at least 10%, still more preferably by at least 30%, still more preferably by at least 50%, still more preferably by at least 70%, even more preferably by at least 90%, may be selected for further testing as a prophylactic and/or therapeutic anti-NASH agent. Sp1 and phosphorylated Sp1 can be determined by conventional techniques, e.g., by Western blotting.

Agents that are found to be capable of decreasing Sp1 activity may be used, for example, to reduce the symptoms of NASH alone or in combination with other appropriate agents or treatments.

In another aspect, the invention relates to the use of a phosphorylated Sp1 protein in a method for screening for, identifying, developing and/or evaluating the efficacy of potentially therapeutic compounds against NASH.

Recently, screening methods based on competitive or non-competitive binding of the molecule with therapeutic potential to the therapeutic target have attracted much attention.

The following example serves to illustrate the invention.

### EXAMPLE 1

### Chromatin immunoprecipitation assay

### 1.1 Crosslinked chromatin preparation

Liver tissue samples from both control (WT) and MAT1A-KO mice [Lu SC et al. Proc. Natl. Acad. Sci. USA 98:5560-5565 (2001)], at 15 days, 1, 3, 5 and 8 months, were excised and immersed into 10 ml of 1xPBS pH 7.4 and 1% formaldehyde, and gently stirred for 12 minutes at room temperature, in order to crosslink the transcription factors to DNA (crosslinked chromatin). The reactions were stopped by addition of glycine to a final concentration of 0.125 M. The liver tissue samples were washed twice with 10 ml of cold 1xPBS pH 7.4 and immersed into 8 ml of 1x PBS pH 7.4 supplemented with 2 µl/ml of a protease inhibitor cocktail (Sigma). The liver tissues samples were disaggregated with a Dounce homogenizer, passed through a 200 µm pore filter and centrifuge at 1,500 g for 5 minutes. The cell pellets were resuspended in 3 ml of cell lysis buffer (5 mM HEPES pH 8.0, 85 mM KCl, 0.5% NP40) supplemented with a protease inhibitor cocktail (Sigma), incubated on ice for 15 minutes and centrifuged at 3,500 g for 5 minutes to pellet the nuclei. The nuclei pellets were resuspended in nuclear lysis buffer (50 mM Tris HCl pH 8.1, 10 mM EDTA, 1% SDS), supplemented with the same protease inhibitor cocktail mentioned above, at a ratio 1:1 (v:wt) relative to the initial liver tissue weight, incubated on ice for 10 minutes aliquoted in 1 ml fractions and stored at -20°C or at -80°C until use for Sp1-ChIP.

### 1.2 Sp1-ChIP assay

1 ml of each sample of crosslinked chromatin was sonicated on ice with 7 pulses of 10 s and 40% amplitude in a Vibra-Cell VCX-500 sonicator (Sonics and Materials). The average chromatin size of the fragments obtained was about 500 bp. The sonicated chromatin was centrifuged at 14,000 g for 10 minutes at 4°C and the supernatants, containing soluble chromatin fragments, were diluted 10-fold with dilution buffer (165 mM NaCl, 0.01 % SDS, 1.1% Triton X, 1.2 mM EDTA, 16.7 mM Tris HCl, pH 8.0) supplemented with a protease inhibitor cocktail (Sigma). The diluted chromatin fractions were precleared by adding 30 µL/mL of 1:1 (v/v) protein A/G sepharose (Amersham Biosciences) (previously blocked during 1 hours with 100 µg/mL lambda DNA, 500 µg/mL tRNA and 1 mg/mL BSA) and incubated at 4°C for 4 hours in a rotating plate. The suspensions were then centrifuged at 14,000 g for 30 s to discard unbound protein A/G sepharose and non-specifically-bound chromatin fragments and the supernatants were fractioned in aliquots equivalent to 50 µg DNA.

The immunofractionation of complexes was performed by adding to each aliquot 2 µg of an anti-Sp1 antibody (Santa Cruz Biotechnology, Inc., Sp1 sc-59) and incubating at 4°C overnight under rotation in a rotating plate. The samples were then incubated with 50 µl of blocked protein A/G sepharose for 4 hours at 4°C under gentle rotation and the immnunocomplexes, containing chromatin fragments/Sp1-antibody/protcin A/G sepharose, were collected by centrifugation at 14,000 g for 30 s, washed twice with low-salt buffer (150 mM NaCl 0.5% deoxycholate, 0.1% SDS, 1% Nonidet P-40, 1 mM EDTA, 50 mM Tris HCl, pH 8.0), twice with high salt buffer (500 mM NaCl 0.5% deoxycholate, 1% Nonidet P-40, 1 mM EDTA, 50 mmM Tris HCl, pH 8.0), twice with LiCl buffer (250 mM LiCl 0.5% deoxycholate, 0.1% SDS, 1% Nonidet P-40, 1 mM EDTA, 50 mM Tris HCl, pH 8.0) and twice with TE buffer (10 mM Tris HCl, 0.25 mM EDTA) pH 8.0. During each washing, the suspension was kept under rotation for 5 minutes at 4°C. An aliquot of the crosslinked chromatin was treated as above, but in the absence of the antibody (***NoAb* fraction**) and the first supernatant, after the preclearing with protein A/G sepharose, was saved as ***Input* fraction.** The immunselected chromatin was eluted from the protein A/G sepharose by two consecutive extractions with 100 µl of elution buffer (1 % SDS, 100 mM NaHSO₃) each, with 30 s of vigorous shaking in a vortex and centrifugation at 12,000 g for 2 minutes. Both supernatants were combined [immunoprecipitated or ***IP* fraction** (Sp1 in Figure 1)] and incubated at 65°C overnight to reverse formaldehyde crosslinks. The DNA from all the fractions (*NoAb* fraction, *Input* fraction and *IP* fraction) was extracted after protease K incubation and purified with a DNA purification kit (PCR purification kit, Qiagen) and used for PCR analysis of the target genes. Inventors routinely tested the specificity of chromatin immunoprecipitation by checking the presence of Sp1 in the *IP* fraction by western blotting analysis, carried out with the antibody used to immunoprecipitate the chromatin fragments.

### 1.3 PCR analysis of immunoprecipitated chromatin

In order to check if the immunoprecipitated chromatin fraction contained genes with the Sp1 bound to the promoter among the DNA pool, the DNA fractions (*NoAb* fraction, *Input* fraction and *IP* fraction) were analyzed by PCR using specific primers for each gene in order to amplify products of 180-300 bp in length, corresponding to either the promoter or coding regions of the target genes. For the analysis 1:5,000 dilutions of the *Input* fraction and 1:30 of the *IP* fraction and *NoAb* fraction were used. PCR analysis of the samples was done by using the following primers:

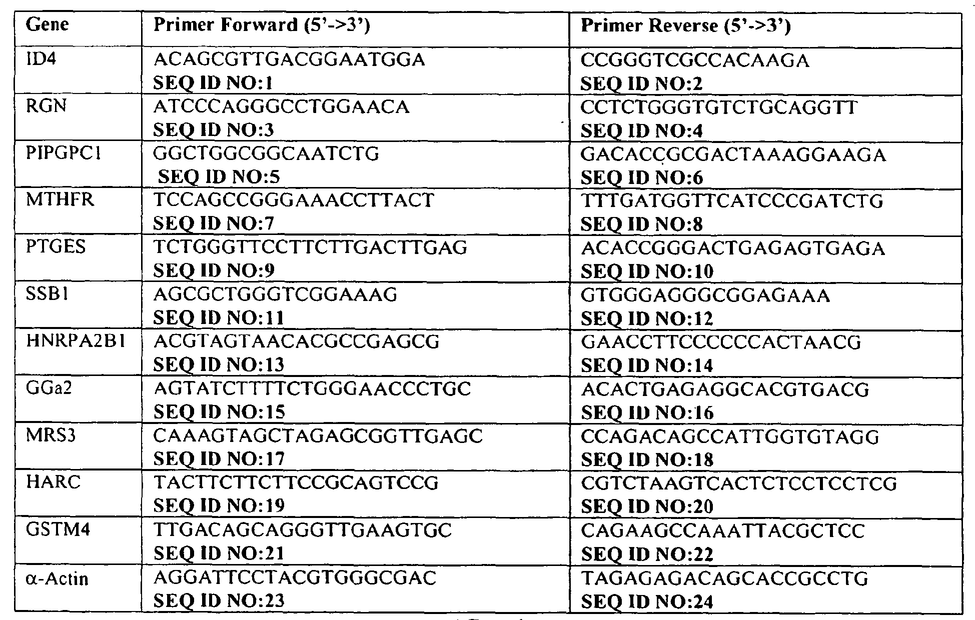

### 1.4 Results

The results obtained are shown in Figure 1. ChIP assay with an anti-Sp1 antibody confirmed that Sp1 binds to the selected gene promoters. From the 11 genes analyzed, the expression of 9 of said genes (ID4, PIGPC1, MTHFR, PTGES, SSB1, HNRPA2B1, MRS3, HARC and GSTM) was enhanced in MAT1A-KO mice liver as compared to the expression thereof in WT mice liver, whereas the expression levels of 2 genes (RGN and GGa2) was similar in livers of WT and MAT1A-KO mice.

### EXAMPLE 2

### Determination of phosphorylated Sp1 in mice liver tissues

### 2.1 Immunoprecipitation (IP) of phosporylated-SP1

Frozen liver tissue samples from control (WT) and MAT1A-KO mice liver, at 15 days, 1, 3, 5 and 8 months, were homogenized in a buffer containing 10 mM Tris C1H pH 7.6, 5 mM EDTA, 50 mM NaCl, 1% Triton X-100, complete protease inhibitor cocktail (Sigma), and 50 mM NaF. The homogenate was centrifuged for 20 minutes at 40,000 g, and supernatants were collected. Protein (500 µg) was immunoprecipitated with 4 µg of anti-Sp1 antibody (Santa Cruz Biotechnology, Inc., Sp1 sc-59) and 20 µl of Protein A Sepharose 4B (Amersham Pharmacia) in binding buffer containing 150 mM NaCl, 10 mM Tris-HCl, pH 7.6, 1 mM EDTA, 1% deoxycholate, 1% NP-40, 0.25 M LiCl. The samples were rotated overnight at 4°C. The immunoprecipitates were pelleted by centrifugation (1,500 g) and washed 3 times with binding buffer.

### 2.2 Western Blotting

Immunoprecipitated complexes were dissolved in SDS buffer, separated by SDS-PAGE (7.5%) and analyzed by immunoblotting using commercial antibodies. The immunoblots were developed with a 1:1,000 dilution of an antibody against phosphoserine (Sigma) or against Sp1 (Santa Cruz Biotechnology, Inc., Sp1 sc-59) and a secondary anti-rabbit or anti-mouse antibody conjugated to horseradish peroxidase (Invitrogen) and the luminal-chemiluminescence reagent (ECL, Amersham Biosciences). The processed blots were exposed to X-ray film, and the autoradiograms were analyzed.

### 2.3 Results

The results of the Western blots are shown in Figure 2. Upper panel clearly shows that the phosphorylation state of Sp1 is increased in liver samples from MAT1A-KO relative to liver samples from WT. Lower panel shows that the amount of total Sp1 screened with anti-Sp1 antibody is the same in MAT1A-KO and WT in the total crude extract. Consequently, an increased level of phosphorylated Sp1 in liver tissue samples relative to the level of phosphorylated Sp1 in control liver tissue samples is indicative of NASH.

### EXAMPLE 3

### Determination of phosphorylated Sp1 in human liver tissues

### 3.1 Immunoprecipitation (IP) of phosporylated-SP1

This assay was carried out with liver tissue samples from:
- normal subjects, i.e., subjects with normal hepatic function with normal liver histology, n=6 (3 female, 3 male; mean age 57.6 years; range 23 - 79 years); and
- NASH diagnosed subjects, namely, NASH grade 1 histologically diagnosed, n=9 (7 female, 2 male; mean age 41.1 years; range 24 - 61 years) [NASH grade 1 was histologically established (macrovcsicular steatosis, lobular, portal inflammation and Mallory bodies) in the absence of other (viral, alcohol, metabolic) causes of NASH [Brunt EM, et al., 1999, Am. J. Gastroenterol. 94, 2467-2474].

Frozen liver tissue samples from both control subjects (i.e., normal subjects) and from NASH diagnosed subjects were homogenized in a buffer containing 10 mM Tris C1H pH 7.6, 5 mM EDTA, 50 mM NaCl, 1% Triton X-100, complete protease inhibitor cocktail (Sigma), and 50 mM NaF. The homogenate was centrifuged for 20 minutes at 40,000 g, and supernatants were collected. Protein (500 µg) was immunoprecipitated with 4 µg of human anti-Sp1 antibody ((H-225):sc-14027, Santa Cruz Biotechnology, Inc) and 20 µl of Protein A Sepharose 4B (Amersham Pharmacia) in binding buffer containing 150 mM NaCl, 10 mM Tris-HCl, pH 7.6, 1 mM EDTA, 1% deoxycholate, 1% NP-40, 0.25 M LiCl. The samples were rotated overnight at 4°C. The immunoprecipitate was pelleted by centrifugation (1,500 *g*) and washed 3 times with binding buffer.

### 3.2 Western Blotting

Immunoprecipitated complexes were dissolved in SDS buffer, separated by SDS-PAGE (7.5%) and analyzed by immunoblotting using commercial antibodies. The immunoblots were developed with a 1:1,000 dilution of an antibody against phosphoserinc (Sigma) or against Sp1 ((H-225):sc-14027, Santa Cruz Biotechnology, Inc) and a secondary anti-rabbit or anti-mouse antibody conjugated to horseradish peroxidase (Invitrogen) and the luminal-chemilumminescence reagent (ECL, Amersham Biosciences). The processed blots were exposed to X-ray film, and the autoradiograms were analyzed.

### 3.3 Results

The results of the Western blots are shown in Figure 3A. Upper panel shows that Sp1 was found to be slightly but significantly (p<0.05) hyperphosphorylated in liver samples from NASH diagnosed subjects as compared to control subjects with normal hepatic function. Lower panel shows that the amount of total Sp1 screened with anti-Sp1 antibody is the same in NASH diagnosed subjects and in control subjects in the total crude extract. Densitometric changes are shown in Figure 3B, expressed as fold of induction of Sp1 phosphorylation over control sample value. Differences between NASH and control were statistically significant, p<0.05- These results confirm that an increased level of phosphorylated Sp1 in human liver tissue samples relative to the level of phosphorylated Sp1 in control human liver tissue samples is indicative of NASH.

## Claims

1. An *in vitro* method to detect non-alcoholic steatohepatitis (NASH) in a subject, or to monitor the effect of the therapy administered to a subject with said condition, which comprises:
a) quantifying the level of phosphorylation of Sp1 protein in a liver tissue sample from said subject; and
b) comparing said level to that of a control sample;
wherein an increase in said level relative to that of the control is indicative of NASH.

2. Method according to claim 1, wherein the sample to be analysed is taken from a subject not previously diagnosed with NASH or from a subject who has been previously diagnosed with NASH, or from a subject receiving anti-NASH treatment, or from a subject who has received anti-NASH treatment previously.

3. Method according to claim 1, which comprises the extraction of the sample to obtain a protein extract.

4. Method according to claim 3, wherein the quantification of the phosphorylation of Sp1 protein comprises contacting the protein extract with a composition of one or more antibodies specific for one or more phospho-serine residues in the phosphorylated Sp1 protein, and quantifying the complexes formed.

5. Method according to claim 4, wherein said antibodies arc monoclonal or polyclonal antibodies, intact or recombinant fragments of antibodies, combibodies and Fab or scFv antibody fragments, specific for the phosphorylated Sp1 protein, whether human, humanised or non-human in origin.

6. Method according to claims 4 or 5, wherein the quantification of said complexes is performed by Western-blotting, ELISA (Enzyme-Linked Immunosorbent Assay), RIA (Radioimmunoassay), Competitive EIA (Competitive Enzyme Immunoassay), DAS-ELISA (Double Antibody Sandwich-ELISA), immunocytochemical or immunohistochemical techniques, techniques based on the use of biochips or protein microarrays that include specific antibodies, assays based on the precipitation of colloidal gold in formats such as dipsticks; or by affinity chromatography techniques, ligand binding assays or lectin binding assays.

7. Use of a phosphorylated Sp1 protein to *in vitro* diagnose NASH in a subject, or to *in vitro* monitor the effect of the therapy administered to a subject with said condition.

8. An *in vitro* method for identifying and/or evaluating the efficacy of a potentially therapeutic agent against NASH, which comprises:
a) contacting a culture of liver cells with a test compound under the appropriate conditions and for the required period of time for them to interact;
b) determining the level of phosphorylation of Sp1 protein;
c) comparing said level obtained in step b) to that of a control culture of liver cells lacking said test compound; and
d) selecting a test compound which causes said level of step b) to decrease.

9. Use of a phosphorylated Sp1 protein in an *in vitro* method for screening for, identifying, developing and/or evaluating the efficacy of potentially therapeutic compounds against NASH.

## Patentansprüche

1. *In* vitro-Verfahren zum Nachweis von nichtalkoholischer Steatohepatitis (NASH) bei einem Individuum oder zur Überwachung der Wirkung der Therapie, die an einem unter diesem Zustand leidenden Individuum verabreicht wurde, umfassend:
a) Quantifizieren des Phosphorylierungsspiegels von Sp1-Protein in einer Lebergewebeprobe des Individuums; und
b) Vergleichen des Spiegels mit dem einer Kontrollprobe;
wobei eine Erhöhung des Spiegels gegenüber dem Spiegel der Kontrolle ein Indikator für NASH ist.

2. Verfahren nach Anspruch 1, wobei die zu untersuchende Probe von einem Individuum stammt, bei dem zuvor NASH nicht diagnostiziert wurde, oder von einem Individuum, bei dem zuvor NASH diagnostiziert wurde, oder von einem Individuum, das gegen NASH behandelt wird, oder von einem Individuum, das zuvor gegen NASH behandelt wurde.

3. Verfahren nach Anspruch 1, das die Extraktion der Probe zum Erhalt eines Proteinextrakts umfasst.

4. Verfahren nach Anspruch 3, wobei die Quantifizierung der Phosphorylierung des Sp1-Proteins das Inkontaktbringen des Proteinextrakts mit einer Zusammensetzung aus einem oder mehreren Antikörpern, die für ein oder mehrere Phosphoserinreste des phosphorylierten Sp1-Proteins spezifisch sind, und die Quantifizierung der gebildeten Komplexe umfasst.

5. Verfahren nach Anspruch 4, wobei die Antikörper monoclonale oder polyclonale Antikörper, intakte oder rekombinante Antikörperfragmente, kombinatorische Antikörper und Fab- oder scFv-Antikörperfragmente, die für das phosphorylierte Sp1-Protein spezifisch sind, ob menschlichen, humanisierten oder nicht-menschlichen Ursprungs.

6. Verfahren nach Anspruch 4 oder 5, wobei die Quantifizierung der Komplexe durchgeführt wird mithilfe von Western-Blotting, ELISA (enzymgekoppelter Immunadsorptionsassay), RIA (Radioimmunassay), kompetitiver EIA (Kompetitiver Enzymimmunassay), DAS-ELISA (Double Antibody Sandwich-ELISA), immuncytochemischen oder immunhistochemischen Verfahren, die auf der Verwendung von Biochips oder Protein-Mikroarrays beruhen, die spezifische Antikörper einschließen, Assays, die auf der Ausfällung von kolloidalem Gold beruhen, in Testformaten wie Dip-Sticks; oder mithilfe von Affinitätschromatographieverfahren, Ligandenbindungsassays oder Lectinbindungsassays.

7. Verwendung eines phosphorylierten Sp1-Proteins zur *in vitro*-Diagnose von NASH bei einem Individuum oder zur *in vitro*-Überwachung der Wirkung der Therapie, die an einem unter diesem Zustand leidenden Individuum verabreicht wurde.

8. *In vitro*-Verfahren zum Identifizieren und/oder Auswerten der Wirksamkeit eines potentiellen Therapeutikums gegen NASH, umfassend:
a) Inkontaktbringen einer Leberzellkultur mit einer Testverbindung unter den geeigneten Bedingungen und über einen Zeitraum, der für eine Wechselwirkung zwischen diesen erforderlich ist;
b) Bestimmen des Spiegels an phosphoryliertem Sp1-Protein;
c) Vergleich des in Schritt b) erhaltenen Spiegels mit dem Spiegel einer Kontrollkultur von Leberzellen ohne die Testverbindung; und
d) Auswählen einer Testverbindung, die ein Absinken des Spiegels aus Schritt b) bewirkt.

9. Verwendung eines phosphorylierten Sp1-Proteins in einem *in* vitro-Verfahren zum Screenen, Identifizieren, Entwickeln und/oder Bewerten der Wirksamkeit potentieller therapeutischer Verbindungen gegen NASH.

## Revendications

1. Procédé *in vitro* pour détecter une stéatohépatite non alcoolique (NASH) chez un sujet, ou pour contrôler l'effet de la thérapie administrée à un sujet ayant ladite affection, qui comprend :
a) la quantification du degré de phosphorylation de la protéine Sp1 dans un échantillon de tissu de foie provenant dudit sujet ; et
b) la comparaison dudit degré avec celui d'un échantillon témoin;
dans lequel un accroissement dudit degré par rapport à celui du témoin est indicatif d'une NASH.

2. Procédé selon la revendication 1, dans lequel l'échantillon destiné à être analysé est prélevé auprès d'un sujet auquel on n'a pas préalablement diagnostiqué une NASH, ou auprès d'un sujet auquel on a préalablement diagnostiqué une NASH, ou auprès d'un sujet recevant un traitement anti-NASH, ou auprès d'un sujet ayant préalablement reçu un traitement anti-NASH.

3. Procédé selon la revendication 1, qui comprend l'extraction de l'échantillon pour obtenir un extrait à base de protéines.

4. Procédé selon la revendication 3, dans lequel la quantification de la phosphorylation de la protéine Sp1 comprend la mise en contact de l'extrait à base de protéines avec une composition à base d'un ou plusieurs anticorps spécifiques d'un ou plusieurs résidus de phosphoserine de la protéine Sp1 phosphorylée, et la quantification des complexes formés.

5. Procédé selon la revendication 4, dans lequel lesdits anticorps sont des anticorps monoclonaux ou polyclonaux, des fragments d'anticorps intacts ou recombinants, des anticorps combinés et des fragments d'anticorps Fab ou scFv, spécifiques de la protéine Sp1 phosphorylée, qu'elle soit humaine, humanisée ou d'origine non humaine.

6. Procédé selon la revendication 4 ou 5, dans lequel la quantification desdits complexes est effectuée par analyse Western blot, ELISA (dosage par immunosorption lié à une enzyme), RIA (dosage radioimmunologique), EIA compétitive (dosage immunologique par enzyme concurrente), DAS-ELISA (dosage à deux anticorps en sandwich-ELISA), des techniques immunocytochimiques ou immunohistochimiques, des techniques basées sur l'utilisation de biopuces ou de microréseaux de protéines qui comprennent des anticorps spécifiques, des dosages basés sur la précipitation d'or colloïdal sous des formes telles que des bandelettes réactives ; ou par des techniques de chromatographie d'affinité, des dosages par liaison de ligand ou des dosages par liaison de lectine.

7. Utilisation d'une protéine Sp1 phosphorylée pour diagnostiquer, *in vitro*, une NASH chez un sujet ou pour contrôler, *in vitro,* l'effet de la thérapie administrée à un sujet ayant ladite affection.

8. Procédé *in vitro* pour identifier un agent thérapeutique potentiel contre la NASH et/ou évaluer son efficacité, qui comprend :
a) la mise en contact d'une culture de cellules hépatiques avec un composé de réaction dans les conditions appropriées et pendant la période de temps requise pour que ceux-ci interagissent ;
b) la détermination du degré de phosphorylation de la protéine Sp1 ;
c) la comparaison dudit degré obtenu dans l'étape b) avec celui d'une culture témoin de cellules hépatiques, exempte dudit composé de réaction ; et
d) la sélection d'un composé de réaction qui provoque la diminution dudit degré de l'étape b).

9. Utilisation d'une protéine phosphorylée Sp1 dans un procédé *in vitre* de criblage pour l'identification, le développement et/ou l'évaluation de l'efficacité de composés potentiellement thérapeutiques contre la NASH.
